(19) **Europäisches Patentamt**
**European Patent Office**
**Office européen des brevets**

(11) **EP 4 740 971 A1**

(12) **EUROPEAN PATENT APPLICATION**

(43) Date of publication:
**13.05.2026 Bulletin 2026/20**

(21) Application number: **25214985.1**

(22) Date of filing: **11.11.2025**

(51) International Patent Classification (IPC):
**A61L 27/42** (2006.01)       **B33Y 70/10** (2020.01)
**B33Y 80/00** (2015.01)       **A61L 27/54** (2006.01)

(52) Cooperative Patent Classification (CPC):
**A61L 27/427; A61L 27/54; B33Y 10/00;**
**B33Y 70/00; B33Y 70/10; B33Y 80/00;**
A61L 2300/102; A61L 2300/404; A61L 2430/12;
A61L 2430/24

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH LA MA MD TN**

(30) Priority: **12.11.2024 US 202463719217 P**

(71) Applicant: **Innojet Technology Co., Ltd.**
**Taoyuan City 320017 (TW)**

(72) Inventors:
• **CHANG, Jen-Hsien**
  **320017 Taoyuan-City (TW)**
• **TANG, Wei-Cheng**
  **320017 Taoyuan-City (TW)**
• **HUANG, Yang-Sheng**
  **320017 Taoyuan-City (TW)**
• **LIU, Hsi-Sheng**
  **320017 Taoyuan-City (TW)**

(74) Representative: **J A Kemp LLP**
**80 Turnmill Street**
**London EC1M 5QU (GB)**

(54) **ADDITIVE MANUFACTURING ANTIBACTERIAL COMPOSITE MATERIAL AND APPLICATION AND PREPARATION PROCESS THEREOF**

(57)     The present disclosure relates to antibacterial composite materials made by additive manufacturing (AM), and application and preparation process thereof.

EP 4 740 971 A1

Description

## TECHNICAL FIELD OF THE INVENTION

The present disclosure relates to antibacterial composite materials made by additive manufacturing (AM), and application and preparation processes thereof.

## BACKGROUND OF THE INVENTION

Medical implant components are used in various clinical and healthcare applications to provide benefits during or after medical treatments. Developments in material science and medical sciences continue to benefit patients receiving surgery or implants. As an example, artificial joints, which can improve motor function for those suffering from age-related conditions or diseases, and may prolong the active lifetime of patients.

However, concerns regarding prognoses of surgery or medical implants remain, in particular with bacterial infection or related implications, which can warrant additional surgery or even lead to failure and retrieval of implants.

Hence, there is a need for novel and cost-effective antibacterial composite materials, in particular for medical implants, and processes for preparation of the same.

## SUMMARY OF THE INVENTION

The present disclosure thus relates to an additive manufacturing antibacterial composite material, comprising:

(a) a substrate of metal or alloy comprising at least one metal selected from the group consisting of titanium (Ti), zirconium (Zr), iron (Fe), cobalt (Co), copper (Cu) and magnesium (Mg);
(b) an inorganic carrier comprising a material selected from the group consisting of phosphates of an alkali earth metal(s) or a metal of Group 4 (IVB), carbonates of an alkali earth metal(s), sulfates of an alkali earth metal(s), zeolites, bentonites, diatomaceous earth, bioglass and any mixtures thereof, and
(c) an antibacterial metal in an elemental or ionic form and selected from the group consisting of silver (Ag), copper (Cu), zine (Zn), cobalt (Co), chromium (Cr), iron (Fe), manganese (Mn), nickel (Ni), tantalum (Ta) and any mixtures thereof in a non-alloy form, wherein the antibacterial metal is loaded within the inorganic carrier, and the inorganic carrier is presented within or embedded in the substrate metal or alloy.

The present disclosure also relates to use of the composite material in an implant component.

The present disclosure also relates to a method of preparing an additive manufacturing antibacterial composite material, the composite material comprising:

(a) a substrate of a substrate metal or alloy comprising at least one metal selected from the group consisting of titanium (Ti), zirconium (Zr), iron (Fe), cobalt (Co), copper (Cu) and magnesium (Mg);
(b) an inorganic carrier comprising a material selected from the group consisting of phosphates of an alkali earth metal(s) or a metal of Group 4 (IVB), carbonates of an alkali earth metal(s), sulfates of an alkali earth metal(s), zeolites, bentonites, diatomaceous earth, bioglass and any mixtures thereof, and ;
(c) an antibacterial metal, in an elemental or ionic form and selected from the group consisting of silver (Ag), copper (Cu), zine (Zn), cobalt (Co), chromium (Cr), iron (Fe), manganese (Mn), nickel (Ni), tantalum (Ta) and any mixtures thereof in a non-alloy form, the method comprising:

(i) providing particles of the substrate metal or the substrate alloy, particles of the inorganic carrier, and particles of the antibacterial metal;
(ii) conducting powder bed fusion on the particles by selectively melting designated areas of the particles and fusing the particles into a solid layer; and
(iii) repeating steps (i) and (ii) to build the additive manufacturing antibacterial composite material, layer-by-layer according to a digital design. The thus-prepared additive manufacturing antibacterial composite material is characterized in that, the antibacterial metal is loaded within the inorganic carrier, and the inorganic carrier is presented within or embedded in the substrate metal or alloy.

In one embodiment, the antibacterial metal in combination with the inorganic carrier are presented in an amount of at least 0.1 wt.%, e.g., at least 0.2 wt.%, at least 0.3 wt.%, at least 0.4 wt.%, at least 0.5 wt.%, at least 0.75 wt.%, at least 1 wt.%, at least 2 wt.%, at least 3 wt.%, at least 4 wt.%, or at least 5 wt.%, based on the total weight of the composite material.

In any preceding embodiment, the antibacterial metal is presented in an amount of at least 0.005 wt.%, e.g., at least 0.01 wt.%, at least 0.02 wt.%, at least 0.03 wt.%, at least 0.04 wt.%, at least 0.05 wt.%, at least 0.075 wt.%, at least 0.1 wt.%, at least 0.2 wt.%, at least 0.3 wt.%, at least 0.4 wt.%, or at least 0.5 wt.%, based on the total weight of the composite material.

In any preceding embodiments, the antibacterial metal is presented within the inorganic carrier. In any preceding embodiments, the inorganic carrier is presented within and embedded in the substrate metal or alloy.

In any preceding embodiments, examples of phosphates include, but are not limited to, apatites (e.g., hydroxy-/chloro-/fluoro-apatites) or pure phosphate, preferably calcium phosphate, calcium hydroxyapatite or zirconium phosphate. In any preceding embodiments, a metal of Group 4 (IVB) is zirconium (Zr).

In any preceding embodiments, the substrate is porous. In any preceding embodiments, the inorganic carrier is porous. In any preceding embodiments, the composite material is porous. In any preceding embodiments, the porosity of the substrate, inorganic carrier or composite material is about at least 0.1 %, for example at least 0.2%, at least 0.3%, at least 0.4%, at least 0.5%, at least 0.75%, at least 1%, at least 1.5%, at least 2% or at least 2.5%.

In any preceding embodiments, the antibacterial metal in an ionic form is loaded in the inorganic carrier in the absence of ionic bonding between the antibacterial metal and the inorganic carrier. In any preceding embodiments, the antibacterial metal in an ionic form is loaded in the inorganic carrier via electrostatic force.

In any preceding embodiments, the antibacterial metal in an ionic form is formed *in situ* during the additive manufacturing process.

In any preceding embodiments, the antibacterial metal in combination with the inorganic carrier is presented in an amount of at least 0.1 wt.%, e.g., at least 0.2 wt.%, at least 0.3 wt.%, at least 0.4 wt.%, at least 0.5 wt.%, at least 1 wt.%, at least 2 wt.%, at least 3 wt.%, at least 3.5 wt.%, at least 4 wt.%, at least 5 wt.%, or at least 7.5 wt.%, based on the total weight of the composite material.

In any preceding embodiments, relative density of the composite material of the antibacterial composite material components (a), (b) and (c) is at least 20%; the relative density can be, for example, obtained by calculating the ratio of the area of pores to the total area, based on imaging.

In any preceding embodiments, the substrate is Ti, Ti-Ni alloy, Zr alloy, stainless steel, cobalt-chromium- (Co-Cr) alloy or Ti-6Al-4V alloy.

In any preceding embodiments, the inorganic carrier does not exhibit or provide an antibacterial effect.

In any preceding embodiments of the use, the implant component is a part of or the entirety of an artificial joint, an insert associated with an artificial joint, or a temporary anchorage device; cosmetic implants such as facial implants, cranioplasty plates; cardiovascular implants such as pacemakers, stents, and artificial heart valves; or dental implants, etc.

In any preceding embodiments of the process, the period of the composite materials (a), (b) and (c) being presented in a molten form and (a), (b) and (c) being presented in a solid form is controlled to reduce the oxidation of the materials. In one embodiment, the period is controlled by adjusting the retention time of laser to be up to 40 $\mu$s, e.g., up to 37 $\mu$s, up to 35 $\mu$s, up to 30 $\mu$s. The energy density should be sufficient for melting the materials.

In any preceding embodiments of the process, the inorganic carrier does not exhibit or provide an antibacterial effect.

In any preceding embodiments of the process, additive manufacturing is conducted with powder bed fusion; the heating source may include, but is not limited to, laser.

## DETAILED DESCRIPTION OF THE INVENTION

In order to facilitate the understanding of the disclosure herein, terms as used herein are hereby defined as follows.

In the context of the specification and the claims, the singular forms "a", "an" and "the" include plural referents, unless specifically indicated otherwise. Unless otherwise stated, any and all examples or exemplary language (e.g., "such as") provided herein are merely used for better illustration of the present disclosure, instead of limiting the scope of the present disclosure.

It is to be understood that any numerical range recited in this specification is intended to include all sub-ranges encompassed therein. For example, a range from "50 to 70°C" includes all sub-ranges and specific values between the stated minimum value of 50°C and the stated maximum value of 70°C, inclusive, e.g., from 58°C to 67°C, and from 53°C to 62°C, 60°C or 68°C. Since the numerical ranges disclosed are continuous, they contain each numerical value between the minimum and maximum value. Unless otherwise specified, the various numerical ranges indicated in this specification are approximate.

In the present disclosure, the term "about" refers to an acceptable deviation of a given value measured by a person of ordinary skill in the art, depending, in part, on how to measure or determine the value.

In the present disclosure, the term "alkali earth metal(s)" refers to the elements of Group 2 in the periodic table.

In the present disclosure, the term "zeolite(s)" refers to microporous, crystalline aluminosilicate materials, which may be expressed by a general formula "$M^{n+}_{1/n}(AlO_2)^-(SiO_2)_x \cdot yH_2O$," wherein $M^{n+}_{1/n}$ is either a metal ion or $H^+$, preferably M is H, Na or K.

In the present disclosure, the term "bentonite(s)" refers to a clay mainly consisting of montmorillonite (sheet silicates).

In the present disclosure, the term "diatomaceous earth" refers to a naturally occurring, soft, siliceous sedimentary rock, which mainly consists of the fossilized remains of diatoms; the chemical constitution mainly includes about 80 to 90% silica, with about 2 to 4% alumina and about 0.5 to 2% iron oxide.

In the present disclosure, the terms "bioglass" and "bioactive glass" may be interchangeable and refer to surface reactive glass-ceramic biomaterials, which are considered biocompatible to human, and the chemical constitution mainly includes silicate, calcium oxide, sodium oxide and/or phosphorous pentoxide, and optionally other metal oxides such as potassium oxide, magnesium oxide, etc.

In the present disclosure, the term "biocompatible" or "biocompatibility" means having an ability to be in contact with a living system without producing an adverse effect, e.g., (severe) allergic response, damage to cells, tissues or organs in a living body, etc.

In the present disclosure, the term "antibacterial metal" refers to those metals or metal ions exhibiting antibacterial effects.

In the present disclosure, the term "porosity" refers to the level of pore space in a material.

In the present disclosure, the "relative density" has a specific meaning under the context of 3D printing and powder metallurgy, and it refers to the ratio of the actual density of a material to its theoretical maximum density. To determine the relative density of a metal sample, the following steps are typically involved: (a) the sample is sanded with abrasive paper (from 80 to 4,000) and polished using 0.05 um $Al_2O_3$; (b) a high-resolution micrograph of the sample is captured using Hitachi TM4000Plus; (c) the captured image is analyzed using ImageJ software; by carefully selecting appropriate threshold values, the pores within the microstructure can be accurately identified; (d) the built-in functions of ImageJ are utilized to determine the total black area within the micrograph, which corresponds to the porosity of the sample; and the relative density is calculated using the formula:

$$\text{Relative Density} = 1 - \text{Porosity}.$$

A common cause of infection in implant surgery is abundant reproduction of bacteria adhering to the surface of the implant. Resulting severe complications can require additional surgery or removal of the implant, leading to surgical failure and increasing patient difficulty. To reduce bacterial reproduction, imparting antibacterial properties to implants has become an important medical issue.

Antibacterial materials can be organic or inorganic. The former is prone to microbial and poor heat resistance, so more attention and application have been devoted to inorganic antibacterial materials. Commonly, metal ions are adsorbed into the surface of bacterial cell walls to react with the functional groups thereof, affecting the structure or function of microbial cells and achieving bacteriostatic effect.

Currently, antibacterial metal ions can attach to the surface via electroplating, ion implantation, chemical deposition, or coating to form an antibacterial layer. However, shortcomings still need be overcome. For example, porous support or structure may be immersed into hydrogels comprising organic bioactive agents such as antibiotics and the hydrogels can be fixed on the support or structure. However, hydrogels, when being placed in organisms, are influenced by various environmental factors, such as temperature, pH, enzymes, etc., making them prone to degradation or denaturation and affecting the release effectiveness of antibiotics. Another approach is to anodize a bioactive substrate such as titanium alloy to form a layer of $TiO_2$, e.g., in the form of nanotubes, with the treated substrate immersed in an antibacterial metal-containing solution (e.g., silver ions) to allow the metal ions to attach and form an antibacterial surface. However, since the surface treatment gives an oxide layer, under the influence of loading and friction, the limited bonding strength between the oxide layer and the metal body, or the impact of material brittleness may cause the antibacterial layer to peel off or fracture, resulting in premature loss of antibacterial effectiveness. Direct addition/mixing of the antibacterial metal (e.g., copper) and metal substrate, followed by an anodizing treatment and high-temperature nitridization may provide an antibacterial alloy substrate having a porous structure on the surface and the antibacterial metal ions can be released to exhibit an antibacterial effect. Nevertheless, the antibacterial metals in alloys do not possess ionic bonds or polarity, resulting in a weaker ability to release ions in an aqueous solution (e.g., biological conditions), and thus may reduce the antimicrobial effectiveness.

The present disclosure thus provides additive manufacturing antibacterial composite materials, applications thereof, and manufacturing process of the same to overcome these problems.

**Additive manufacturing antibacterial composite materials**

The additive manufacturing antibacterial composite material comprises (a) a substrate of metal or alloy, (b) an inorganic carrier, and (c) an antibacterial metal, wherein the antibacterial metal is loaded within the inorganic carrier presented within or embedded in the substrate metal or alloy. Details of the components and structure are as follows.

(a) substrate metal or alloy

The antibacterial composite materials in the present disclosure are prepared via additive manufacturing (AM). Various metal and non-metal materials can be used in AM processes, and those being biocompatible are used herein, in particular, metals such as titanium (Ti), zirconium (Zr), iron (Fe), cobalt (Co), copper (Cu), magnesium (Mg) or alloys thereof. Preferably, Ti, Ti alloys (e.g., Ti-6Al-4V, Ti6242, etc.), Zr alloys (e.g., Zr-Cu-Al-Nb, Zr-Cu-Ni-Al-Ti, Zr-Cu-Ni-Al-Nb), stainless steel, Co alloys (e.g., Co-Cr-Mo, Co-Cr-W, etc.) are used.

Without being bound to theory, the substrate metals or alloys are not alloyed with the antibacterial metal used herein, or are alloyed at a minimum or trace level, to avoid hindering the release of the antibacterial metal.

(b) inorganic carriers

In the present disclosure, the inorganic carrier is used for carrying (loading) the antibacterial materials and presented in the substrate metal or alloy and/or embedded in the substrate metal or alloy. The inorganic carrier can also prevent or hinder oxidation/reduction reaction or alloy between the antibacterial metal and the substrate metal/alloy. In addition, the inorganic carrier is advantageous in the release of antibacterial metal over existing antibacterial composite materials of implants. For example, in one embodiment, the antibacterial metal in an ionic form is loaded in the inorganic carrier in the absence of ionic bonding between the antibacterial metal and the inorganic carrier. In any preceding embodiments, the antibacterial metal in an ionic form is loaded in the inorganic carrier via electrostatic force. In another embodiment, the inorganic carrier is presented within the substrate metal or alloy. In yet another embodiment, the inorganic carrier is presented within and embedded in the substrate metal or alloy.

In particular, phosphates of an alkali earth metal or a metal of Group 4 (IVB), carbonates of an alkali earth metal, sulfates of an alkali earth metal(s) or an alkali metal(s), zeolites, bentonites, diatomaceous earth, bioglass and any mixture thereof, can be used as the inorganic carrier herein.

Examples of phosphates include, but are not limited to, apatites (e.g., hydroxy-/chloro-/fluoro-apatites) or pure phosphates (e.g., those without hydroxy/chloro/flouro in the crystals). Examples of alkali earth metal(s) include, but are not limited to, magnesium (Mg) and calcium (Ca), preferably Ca. The metal of Group 4 (IVB) is preferably zirconium (Zr). Examples of phosphates of an alkali earth metal(s) or a metal of Group 4 (IVB) include, but are not limited to, calcium phosphate, calcium hydroxyapatite, or zirconium phosphate.

Examples of carbonates of an alkali earth metal include, but are not limited to, calcium carbonate.

Examples of sulfates of an alkali earth metal(s) or an alkali metal(s), include, but are not limited to, sodium sulfate, potassium sulfate, magnesium sulfate and calcium sulfate, preferably calcium sulfate.

Examples of zeolites include, but are not limited to, LTA (Linde Type A), MOR-type, HEU-type and ANA-typ.

Examples of bentonites include, but are not limited to, Na-montmorillonite and Ca-montmorillonite.

Examples of bioglass include, but are not limited to, Bioglass 45S5 or derivative forms of Bioglass 45S5, e.g., having different $CaO/P_2O_5$ ratios.

In one embodiment, the inorganic carriers are presented in an amount of no greater than 1.0 wt.%, e.g., no greater than 0.8 wt.%, no greater than 0.6 wt.%, no greater than 0.5 wt.%, no greater than 0.4 wt.%, no greater than 0.3 wt.%, based on the total weight of the composite material.

(c) antibacterial metals

Antibacterial metals exhibit strong inhibition of adhesion and growth and/or proliferation of bacteria. In the present disclosure, antibacterial metals are in elemental or ionic, rather than alloy, form.

Examples of antibacterial metal include, but are not limited to, silver (Ag), copper (Cu), zine (Zn), cobalt (Co), chromium (Cr), iron (Fe), manganese (Mn), nickel (Ni), tantalum (Ta), and ionic form(s) thereof, preferably Ag, Cu, Zn, Co and ionic form(s) thereof, and any mixtures thereof in non-alloy form.

In one embodiment, the antibacterial metal is at least presented in an amount to exhibit the antibacterial effect, e.g., of at least 0.005 wt.% (50 ppm), e.g., at least 0.01 wt.% (100 ppm), at least 0.1 wt.%, at least 0.2 wt.%, at least 0.3 wt.%, at least 0.4 wt.%, at least 0.5 wt.%, at least 0.75 wt.%, at least 1 wt.%, at least 2 wt.%, at least 3 wt.%, at least 4 wt.%, at least 5 wt.%, based on the total weight of the composite material.

In one embodiment, the antibacterial metal in an ionic form is formed *in situ* during the additive manufacturing process. In one embodiment, the antibacterial metal in an ionic form is released under specific conditions, e.g., biological conditions.

In one embodiment, the antibacterial metal in combination with the inorganic carrier is presented in an amount of at least 0.1 wt.%, e.g., at least 0.2 wt.%, at least 0.3 wt.%, at least 0.4 wt.%, at least 0.5 wt.%, at least 1 wt.%, at least 2 wt.%, at least 3 wt.%, at least 3.5 wt.%, at least 4 wt.%, at least 5 wt.%, at least 7.5 wt.%, based on the total weight of the composite material.

In one embodiment, the weight ratio of the antibacterial metal to the inorganic carrier is no greater than 0.03, e.g., no greater than 0.025, no greater than 0.02, no greater than 0.019, no greater than 0.018, or no greater than 0.015. Without being bound to theory, this weight ration may reflect the efficacy or limitation of protection of the inorganic carrier.

In one embodiment, the antibacterial metal comprises less than 0.05 molar %, preferably less than 0.025 molar% of its oxide form. Without being bound to theory, the release of an oxide form of the antibacterial metal may be slower than that of the antibacterial metal in an elemental form or in an ionic form, in particular under certain specific conditions.

In one embodiment, relative density of the composite material to the components (a), (b) and (c) is at least 20%.

The antibacterial composite materials are prepared via additive manufacturing processes and details are described herein.

In one embodiment, the component (b) is in an amount of 0.05 wt.% to 0.3 wt.% and the component (c) is in an amount of 0.005 wt.% (50 ppm) to 0.015 wt.% (150 ppm), based on the total weight of the composite material. In any previous embodiment, the component (c) is silver. In any preceding embodiment, the component (b) is bioglass. In any preceding embodiment, the component (a) is titanium or titanium alloy.

## Additive manufacturing processes for manufacturing antibacterial composite materials

Traditional powder metallurgy, metal injection molding (MIM), binder jetting, and material extrusion modeling (MEX) can all be used to manufacture metal and non-metal composites. The common feature of these processes is the use of a binder (usually organic) to preform the powder, followed by debinding at high temperatures to remove the binder, and then sintering the metal particles together while retaining the non-metal in the metal substrate. Nevertheless, the sintering process is typically more complex and the finished product usually has lower relative density (e.g., 85 to 93%) than additive manufacturing products, resulting in decreased mechanical strength.

Hence, the present disclosure provides a method of preparing an additive manufacturing antibacterial composite material, the composite material comprising :

(a) a substrate of a substrate metal or alloy comprising at least one metal selected from the group consisting of titanium (Ti), zirconium (Zr), iron (Fe), cobalt (Co), copper (Cu) and magnesium (Mg),
(b) an inorganic carrier comprising a material selected from the group consisting of phosphates of an alkali earth metal(s) or a metal of Group 4 (IVB), carbonates of an alkali earth metal(s), sulfates of an alkali earth metal(s), zeolites, bentonites, diatomaceous earth, bioglass and any mixtures thereof, or;
(c) an antibacterial metal in elemental or ionic form and selected from the group consisting of silver (Ag), copper (Cu), zine (Zn), cobalt (Co), chromium (Cr), iron (Fe), manganese (Mn), nickel (Ni), tantalum (Ta) and any mixtures thereof in non-alloy form; and

The method comprises:

(i) providing particles of the substrate metal or the substrate alloy, particles of the inorganic carrier and particles of the antibacterial metal;
(ii) conducting powder bed fusion on the particles by selectively melting designated areas of the particles and fusing the particles into a solid layer, and
(iii) repeating steps (i) and (ii) to build the additive manufacturing antibacterial composite material, layer-by-layer according to a digital design; such that the antibacterial metal is loaded within the inorganic carrier, and the inorganic carrier is presented within or embedded in the substrate metal or alloy.

In one embodiment, step (i) includes ball milling of the particles. In a specific embodiment, the ball milling process comprising (1) sequentially charging a ball mill chamber with the inorganic carrier, substrate metal or alloy powder, and grinding media, (2) rotating the chamber at a controlled speed for a designated period to achieve intimate contact between materials through collisions and shearing, and (3) discharging the homogenized mixture upon reaching the predetermined level of uniformity. In one embodiment, step (i) includes spreading a thin layer of the (fresh) particles on a platform.

In one embodiment, the period of the materials (a), (b) and (c) being presented in a molten form and (a), (b) and (c) being presented in a solid form is controlled to reduce oxidation thereof. Without being bound to theory, controlling the periods of melting/solidification during the process prevents or reduces oxidation-reduction between the inorganic carrier, antibacterial metal, and metal substrate. This allows the inorganic carrier and antibacterial metal to be retained and evenly dispersed within the metal substrate, and even when the composite material is subjected to wear during use, antibacterial properties can still be maintained.

In addition, additive manufacturing processes may provide metal substrates having high specific surface area and porous structures, which lead to increase in the release of metal ions, and the processes can be adjusted to modify or improve the mechanical strength of the product. Last but not least, additive manufacturing processes may reduce tedious post-processing and minimize the risk of bacterial contamination during transfer.

In one embodiment, the antibacterial metal in an ionic form is formed *in situ* during the additive manufacturing process, in particular during melting.

In one embodiment, the inorganic carrier does not exhibit or provide an antibacterial effect.

In one embodiment, the additive manufacturing is conducted with laser powder bed fusion.

In the additive manufacturing process, one or more of the following process parameters can adopted or adjusted:

(1) laser power of 1 W to 300 W, e.g., 5 W to 15 W, 10 W to 250 W, 15 W to 35 W, 50 W to 75 W, about 100 W, 40 W to 180 W, 25 W to 275 W, about 150 W, 60 W to 90 W, 80 W to 200 W, 120 W to 130 W, 20 W to 30 W, 175 W to 225 W, or any single point or a reasonable numeric range constituted by above-mentioned point values, e.g., 275 W to 300 W, 30 W to 80 W, etc.;

(2) scanning rate of 3 m/s or less, e.g., 2.5 m/s or less, 2 m/s or less, 1 m/s or less;

(3) scanning strategy selected from the group consisting of chess, stripes, contour and meander;

(4) pre-heated temperature of a platform in additive manufacturing: 50 °C to 200 °C, e.g., about 175 °C, 65 °C to 70 °C, 60 °C to 120 °C, 55 °C to 100 °C, about 150 °C, 80 °C to 130 °C, or any single point or a reasonable numeric range constituted by above-mentioned point values, e.g., 120 °C to 130 °C, about 80 °C etc.;

(5) thickness of each layer: 20 $\mu$m to 100 $\mu$m, e.g., about 25 $\mu$m, about 50 $\mu$m, about 75 $\mu$m, 30 $\mu$m to 40 $\mu$m, 80 $\mu$m to 90 $\mu$m, 35 $\mu$m to 60 $\mu$m, or any single point or a reasonable numeric range constituted by above-mentioned point values, e.g., 40 $\mu$m to 50 $\mu$m, about 80 $\mu$m, etc.;

(6) oxygen content (v/v): 0.05% to 3% (v/v) of the total volume in the build chamber, e.g., about 0.5%, about 1.5%, about 0.15%, 0.075% to 0.1%, less than 2.5%, 0.25% to 0.75%, less than 2%, or any single point or a reasonable numeric range constituted by above-mentioned point values; and

(7) inert gas flow rate: 0.1 m/s to 3 m/s, e.g., about 1.75 m/s, 0.3 m/s to 0.5 m/s, 1.5 to 2.5 m/s, 1 m/s to 2 m/s, or any single point or a reasonable numeric range constituted by above-mentioned point values.

## Applications of the additive manufacturing antibacterial composite materials

The additive manufacturing antibacterial composite materials man be applied in an implant component, e.g., a part of or the entirety of an artificial joint, an insert associated with an artificial joint, or a temporary anchorage device; cosmetic implants such as facial implants, cranioplasty plates; cardiovascular implants such as pacemakers, stents, and artificial heart valves; or dental implants, etc.

Without being bound to theory, as noted herein, even when the implant component comprising the composite material is subjected to wear during use, antibacterial properties can be maintained and lifetime and durability of the implant component prolonged.

## EXAMPLES

The following examples are provided to illustrate the present invention to those of ordinary skill in the art without limiting the scope thereof.

## Materials, Methodologies and Test Models

Ti64 powders having particle size from 15 to 53 $\mu$m (available from Shandong Lianhong new material Technology CO. LTD) were used. Silver-containing glass powders can be obtained from Ishizuka CO. LTD.

Porosity can be measured by Hitachi TM4000Plus.

Scanning electronic microscopy (SEM) and energy-dispersive X-ray spectroscopy (EDS) were adopted for imaging and elemental analysis for positioning of the materials.

Antibacterial effect was evaluated by JIS Z 2801, and *Staphylococcus aureus* (strain BCRC 10451) was used. Mechanical properties were evaluated based on ASTM E8, including tensile strength, percentage of elongation, etc. Surface characteristics were evaluated by using ULVAC-PHI PHI 5000 Versaprobe II.

Additive manufacturing is conducted by using Trumpf lasers (e.g., Truprint 2000).

**Example**

Ti64 powders were used as the substrate metal/alloy; silver-containing glass powders loaded in an inorganic carrier were used as the antibacterial metal in combination with the inorganic carrier. The powders were mixed and used for additive manufacturing or being processed by arc melting under vacuum. Different proportions or level of the materials were used. The parameters are listed in Table 1 below:

Table 1

| Sample No. | Antibacterial metal (Ag) level | Inorganic carrier level (wt.%) | Processing manner |
|---|---|---|---|
| Sample 1 | 15ppm | 0.3 | PBF |
| Sample 2 | 27ppm | 0.15 | PBF |
| Sample 3 | 36ppm | 0.2 | PBF |
| Sample 4 | 38ppm | 0.3 | PBF |
| Sample 5 | 48ppm | 0.3 | PBF |
| Sample 6 | 54ppm | 0.3 | PBF |
| Sample 7 | 90ppm | 0.5 | PBF |
| Sample 8 | 450ppm | 2.5 | PBF |
| Sample 9 | 54ppm | 0.3 | Vacuum arc remelting |
| Sample 10 | 0.2% | 11 | Vacuum arc remelting |
| Sample 11 | 0.2% | 11 | PBF |
| Control | 0 | 0 | |

The constitution of the powders (wt.%): P(20~30), Al(2~10), Mg(1~5), Na(1~5), Si(0.1~1.0), Ag(1~5), remainder being O.

Characteristics of the products are summarized in Table 2 below:

Table 2

| Sample No.\ Characteristics | 1 | 2 | 3 | 4 | 5 | 6 |
|---|---|---|---|---|---|---|
| Relative density (%) | 99.6 | 99.8 | 99.8 | 99.7 | 99.6 | 99.7 |
| Ultimate tensile stress(MPa) | 1320 | 1280 | 1315 | 1311 | 1379 | 1348 |
| Elongation (%) | 3.4 | 6 | 4.6 | 3.3 | 3.5 | 3.5 |
| Antibacterial efficacy (%) | 0 | <90 | | 76.56 | 62.85 | 96.37 |
| XPS Ag3d peak (eV) | | | | | | |

| Sample No.\ Characteristics | 7 | 8 | 9 | 10 | 11 | Control |
|---|---|---|---|---|---|---|
| Relative density (%) | 99.2 | 97.6 | | | | 99.9 |
| Ultimate tensile stress(MPa) | 1388 | 1413 | | | | 1240 |
| Elongation (%) | 2.2 | 0.8 | | | | 6.8 |
| Antibacterial efficacy (%) | | >99.0 | <90.0 | <90.0 | | 0 |
| XPS Ag3d peak (eV) | | | | 367.0~ 369.0 | 367.0~ 369.0 | |

In order to determine the dispersion of the antibacterial metal/inorganic carrier within the substrate, the sample was polished and ground and examined by SEM; samples at specific locations were selected and images were obtained. Through the contrast between light and dark zones, the distribution of the inorganic carrier can be preliminarily observed and confirmed.

Further analysis of the element distribution was conducted using EDS. Through the element distribution map, the position of the inorganic carrier and whether the antibacterial metal is retained in the inorganic carrier can be confirmed; homogeneity of the material can also be evaluated.

The invention exhibits superior effects over the existing objects, for example, providing sufficient antibacterial effects while maintaining comparable mechanical properties. Without being bond to the theory, in traditional metal forming processes, high-temperature metals have high reactivity, which may reduce the release of antibacterial metals in inorganic oxides through reduction or alloying. However, according to the subject disclosure, by optimizing the process parameters of powder bed laser, the reaction time of antibacterial metals with the metal substrate at high temperatures can be reduced, thereby reducing the reaction at high temperatures. The unalloyed antibacterial metals that are retained can be more easily released to the surface, achieving better antibacterial effects.

A person of ordinary skill in the art of the subject invention should understand that variations and modification may be made to the teaching and the disclosure of the subject invention without departing from the spirit and scope of the subject application. Based on the contents above, the subject application intends to cover any variations and modifications thereof with the proviso that the variations or modifications or their equivalents fall within the scope as defined in the appended claims.

## Claims

1. An additive manufacturing antibacterial composite material, comprising:

   (a) a substrate, made of a substrate metal or alloy comprising at least one metal selected from the group consisting of titanium (Ti), zirconium (Zr), iron (Fe), cobalt (Co), copper (Cu) and magnesium (Mg);
   (b) an inorganic carrier, comprising a material selected from the group consisting of phosphates of an alkali earth metal(s) or a metal of Group 4 (IVB), carbonates of an alkali earth metal(s), sulfates of an alkali earth metal(s), zeolites, bentonites, diatomaceous earth, bioglass and any mixtures thereof;
   (c) an antibacterial metal, which is in elemental or ionic form and selected from the group consisting of silver (Ag), copper (Cu), zine (Zn), cobalt (Co), chromium (Cr), iron (Fe), manganese (Mn), nickel (Ni), tantalum (Ta) and any mixtures thereof in non-alloy form;

   wherein the antibacterial metal is loaded within the inorganic carrier, and the inorganic carrier is presented within or embedded in the substrate metal or alloy.

2. The composite material according to Claim 1, wherein the weight ratio of the antibacterial metal to the inorganic carrier is no greater than 0.03.

3. The composite material according to any of the preceding claims, wherein the antibacterial metal is presented within the inorganic carrier.

4. The composite material according to any of the preceding claims, wherein the substrate is porous.

5. The composite material according to any of the preceding claims, wherein the antibacterial metal in an ionic form is loaded in the inorganic carrier in the absence of ionic bonding between the antibacterial metal and the inorganic carrier.

6. The composite material according to any of the preceding claims, wherein the antibacterial metal in an ionic form is formed *in situ* during the additive manufacturing process.

7. The composite material according to any of the preceding claims, wherein the antibacterial metal is presented in an amount of at least 0.005 wt.% (50 ppm), based on the total weight of the composite material.

8. The composite material according to any of the preceding claims, wherein the antibacterial metal in combination with the inorganic carrier are presented in an amount of at least 0.1 wt.%, based on the total weight of the composite material.

9. The composite material according to any of the preceding claims, wherein relative density of the composite material to the components (a), (b) and (c) is at least 20%.

10. The composite material according to any of the preceding claims, wherein the substrate is made of Ti, Ti alloys (e.g.,

Ti-6Al-4V, Ti6242, etc.), Zr, Zr alloys (Zr-Cu-Al-Nb, Zr-Cu-Ni-Al-Ti, Zr-Cu-Ni-Al-Nb), stainless steel, Co alloys (e.g., Co-Cr-Mo, Co-Cr-W, etc.).

11. The composite material according to any of the preceding claims, wherein the inorganic carrier does not exhibit or provide an antibacterial effect.

12. Use of the composite material according to any of the preceding claims in an implant component.

13. The use according to Claim 12, wherein the implant component is a part of or the entirety of an artificial joint, an insert associated with an artificial joint, or a temporary anchorage device cosmetic implants such as facial implants, cranioplasty plates; cardiovascular implants such as pacemakers, stents, and artificial heart valves; or dental implants.

14. A process of preparing an additive manufacturing antibacterial composite material, the composite material comprising:

(a) a substrate, made of a substrate metal or alloy comprising at least one metal selected from the group consisting of titanium (Ti), zirconium (Zr), iron (Fe), cobalt (Co), copper (Cu) and magnesium;
(b) an inorganic carrier, comprising a material selected from the group consisting of phosphates of an alkali earth metal(s) or a metal of Group 4 (IVB), carbonates of an alkali earth metal(s), sulfates of an alkali earth metal(s), zeolites, bentonites, diatomaceous earth, bioglass and any mixtures thereof;
(c) an antibacterial metal, which is in elemental or ionic form and selected from the group consisting of silver (Ag), copper (Cu), zine (Zn), cobalt (Co), chromium (Cr), iron (Fe), manganese (Mn), nickel (Ni), tantalum (Ta) and any mixtures thereof in non-alloy form;

the method comprising:

(i) providing particles of the substrate metal or the substrate alloy, particles of the inorganic carrier and particles of the antibacterial metal;
(ii) conducting powder bed fusion on the particles by selectively melting designated areas of the particles and fusing the particles into a solid layer; and
(iii) repeating steps (i) and (ii) to build the additive manufacturing antibacterial composite material, layer-by-layer according to a digital design,

such that the antibacterial metal is loaded within the inorganic carrier, and the inorganic carrier is presented within or embedded in the substrate metal or alloy.

15. The process according to Claim 14, wherein the antibacterial metal in an ionic form is formed *in situ* during the additive manufacturing process.

16. The process according to Claim 14 or 15, wherein the period of the materials (a), (b) and (c) being presented in a molten form and the period of the materials (a), (b) and (c) being presented in a solid form is controlled to reduce the oxidation of these materials.

17. The process according to any one of Claims 14 to 16, wherein the inorganic carrier does not exhibit or provide an antibacterial effect.

18. The process according to any one of Claims 14 to 17, wherein the additive manufacturing is conducted with powder bed fusion.

Europäisches
Patentamt
European
Patent Office
Office européen
des brevets

# EUROPEAN SEARCH REPORT

**Application Number**

EP 25 21 4985

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/298523 A1 (CRUDDEN JOSEPH J [US] ET AL) 3 October 2019 (2019-10-03) * claims 1-5 * * example 6 * * paragraph [0038] * | 1-18 | INV.<br>A61L27/42<br>B33Y70/10<br>B33Y80/00<br>A61L27/54 |
| | ----- | | |
| A | WO 2020/161239 A1 (UNIV MAASTRICHT [NL]; ACAD ZIEKENHUIS MAASTRICHT [NL]) 13 August 2020 (2020-08-13) * page 11 - page 14 * * page 11, line 28 - line 31 * * page 13, line 22 - line 25 * * figure 1 * | 1-18 | |
| | ----- | | |
| A | EP 1 911 468 A2 (HOWMEDICA OSTEONICS CORP [US]) 16 April 2008 (2008-04-16) * paragraph [0001] - paragraph [0002] * * paragraphs [0023], [0029] * | 1-18 | |
| | ----- | | |
| | | | **TECHNICAL FIELDS SEARCHED (IPC)**<br><br>A61L<br>B33Y |

The present search report has been drawn up for all claims

| Place of search | Date of completion of the search | Examiner |
|---|---|---|
| The Hague | 12 March 2026 | Lopez Serrano, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 21 4985

This annex lists the patent family members relating to the patent documents cited in the above-mentioned European search report.
The members are as contained in the European Patent Office EDP file on
The European Patent Office is in no way liable for these particulars which are merely given for the purpose of information.

12-03-2026

| Patent document cited in search report | Publication date | Patent family member(s) | Publication date |
|---|---|---|---|
| US 2019298523 A1 | 03-10-2019 | US 2019298523 A1<br>US 2020315802 A1 | 03-10-2019<br>08-10-2020 |
| WO 2020161239 A1 | 13-08-2020 | NONE | |
| EP 1911468 A2 | 16-04-2008 | AT E444085 T1<br>EP 1911468 A2<br>US 2008050412 A1 | 15-10-2009<br>16-04-2008<br>28-02-2008 |

EPO FORM P0459

For more details about this annex : see Official Journal of the European Patent Office, No. 12/82